# EUROPÄISCHE PATENTSCHRIFT

(11) **EP 1 072 714 B1**
(45) Veröffentlichungstag und Bekanntmachung des Hinweises auf die Patenterteilung: **21.07.2004**
(21) Anmeldenummer: 00120798.4
(22) Anmeldetag: 11.01.1989
(51) Int. Cl.: D06F 39/02

(54) **Verfahren zum Wäsche waschen**
Method for washing the laundry
Procédé pour laver le linge

(30) Priorität: 15.02.1988 DE 8801919 U; 12.10.1988 DE 8812795 U
(43) Veröffentlichungstag der Anmeldung: 31.01.2001
(62) Teilanmeldung aus: 89100395.6
(73) Patentinhaber: Henkel Kommanditgesellschaft auf Aktien, 40191 Düsseldorf (DE)
(72) Erfinder: Weber, Rudolf, 40589 Düsseldorf (DE); Jobs, Jörn, 41749 Viersen (DE)
(74) Vertreter: Müller, Enno, Dipl.-Ing.

(56) Entgegenhaltungen:
- GB-A- 302 416
- GB-A- 1 298 454
- US-A- 3 048 993
- US-A- 3 400 808

## Beschreibung

Die Erfindung betrifft ein Verfahren zum Wäsche waschen in einer Waschmaschine, bei welchem man eine sackartige Vorrichtung verwendet, welche eine geschmeidige Hülle aufweist, welche Hülle mit der gewünschten Menge eines eine bestimmte Aktivität im Verlaufe des Waschvorganges entfaltenden, teilchenförmigen Produktes gefüllt ist, wobei das Material, aus welchem diese Hülle besteht, das Eindringen des wässrigen Waschmediums in den Sack ermöglicht, und wobei die Vorrichtung zusammen mit der Wäsche in die Waschmaschine eingebracht wird.

Bei der Verwendung von Flüssigwaschmittel ist man dazu übergegangen, dieses dosiert in ein Speichergefäß einzufüllen, das mit der zu behandelnden Wäsche in die Trommel einer Waschmaschine eingebracht wird. Durch die Rotation der Trommel tritt während des Waschvorganges das Flüssig-Waschmittel aus einer Austrittsöffnung des Speicherbehälters aus. Der Speicherbehälter besteht aus starrem Kunststoffmaterial. Bei nicht geeigneter Verfahrensweise kann es vorkommen, dass eine Entleerung des Speicherbehälters gleich zu Beginn des Waschvorganges - wenn noch kein oder nur wenig Wasser in der Waschmaschine vorhanden ist - oberhalb des Laugenstutzens der Waschmaschine erfolgt, so dass das Flüssig-Waschmittel in den Laugenstutzen eintritt und für den Waschvorgang nicht mehr oder nur teilweise zu Verfügung steht.

Die US-A-3,947,971 beschreibt eine Verfahrensweise betreffend eine Dosiervorrichtung mit einem Paar flexibler, poröser, offenzelliger Schaumstoffplatten, welche aufeinanderliegend derart miteinander befestigt sind, dass ein zu einem Rand hin unverschlossener Spalt ausgebildet ist. In diesen Spalt wird ein Wäschebehandlungsmittel in Form einer Tablette unter Öffnung des Spaltes eingeführt und diese Dosiervorrichtung dann in dieser Form in die Waschmaschine eingegeben.

Aus der US-A 4,532,722 ist eine Vorrichtung zur Abgabe eines Konditionierungsmittels für Textilien in einem Wäschetrockner bekannt. Eine solche Vorrichtung ist mit einem absorbierenden Material gefüllt, das weiter mit einem flüssigen Textil-Konditionierungsmittel bis zur Sättigung übergossen wird. Das Konditionierungsmittel soll nur dadurch abgegeben werden, dass Heißluft, die den Wäschetrockner durchsetzt, zur Verdampfung des Konditionierungsmittels führt. Der Dampf erst tritt dann durch Öffnungen in dem Behältnis aus, um die gewünschten Duft-Eigenschaften auf das Textil zu übertragen. Zu diesem Zweck ist die Wandung des Behältnisses aus einem lediglich für Wasserdampf, nicht aber für Wasser durchlässigen Textil gebildet.

Die GB-A-302 416 betrifft eine Vorrichtung zur Zugabe von einem Färbemittel zu dem Wasser, in welchem weiße Kleidungsstücke gewaschen werden.

Der Erfindung liegt die Aufgabe zugrunde, eine Dosiervorrichtung der eingangs genannten Art zu schaffen, bei der, unter Vermeidung der genannten Nachteile, Verluste von Waschmittelsubstanz vermieden werden. Gleichzeitig soll die Möglichkeit gegeben sein, mit den gleichen Dosiergefäßen pulver- oder pastenförmige Produkte verlustfrei zu dosieren.

Hiervon ausgehend beschäftigt sich die Erfindung mit der Aufgabe, ein Verfahren zum Wäsche waschen in einer Waschmaschine anzugeben, das zu vorteilhaften Ergebnissen führt.

Diese Aufgabe ist bei den Gegenständen der Ansprüche 1 und 2 der Anmeldung gelöst.

Bei Verwendung einer den Speicher beinhaltenden, verschließbaren Hülle aus offenporiger Zellstruktur kann auch bei unsachgemäßer Vorgehensweise das im Speicher befindliche Waschmittel oder dergleichen, aufgrund der Rückhaltefähigkeit einer derartigen Hülle, nicht direkt in umnfangreicher Menge austreten. Diese Rückhaltefähigkeit resultiert aus einer gewissen Rückhaltewirkung der Zellstruktur gegenüber dem Wäschebehandlungsmittel. Das Wasser hingegen kann jedoch die Zellstruktur leicht durchdringen und dann das verdünnte bzw. gelöste Wäschebehandlungsmittel aus der Hülle herausspülen und/oder die Anordnung ist so getroffen, dass die Wäschebehandlungspaste aufgrund ihrer relativen Dickflüssigkeit nur langsam die Zellstruktur durchdringt (hindurchdiffundiert), so dass über einen langen Zeitraum kontinuierlich das Behandlungsmittel an das Waschwasser abgegeben wird, was äußerst förderlich für das Waschergebnis ist. Es ist damit stets sichergestellt, dass erst mit Zulauf des Waschwassers ein nennenswertes Austreten des Wäschebehandlungsmittels aus der erfindungsgemäßen Vorrichtung erfolgt. Insbesondere ist die Zellstruktur flexibel ausgebildet, indem sie vorzugsweise aus einem textilen Flächengebilde besteht, wodurch sich eine hohe Wäschefreundlichkeit und ein "geräuschloser" Waschvorgang ergibt. Die letzteren Eigenschaften sind bei den bekannten aus festem Kunststoff bestehenden Dosiervorrichtungen nicht gegeben, da beim Waschvorgang eine erhebliche Reibung an dem harten Speicherbehälter auftritt und der Behälter beim Anstoßen an die Trommelwandungen ein störendes Geräusch auslöst.

Nach einer Weiterbildung der Erfindung ist vorgesehen, dass die Hülle als Beutel ausgebildet ist.

Alternativ kann jedoch auch vorgesehen sein, dass die Hülle als kuvertartige Tasche ausgebildet ist.

Um die hohe Flüssigkeitsrückhaltefähigkeit und auch Wäschefreundlichkeit zu erzielen, besteht die Hülle vorzugsweise aus Gewebe, Gewirke oder Vlies. Möglich ist es bevorzugt ferner, Schlingenware, z.B. Frottier oder Frottee, Strickware, Damast oder Schaumstoff zu verwenden.

Für den Verschluss der Hülle besteht die Möglichkeit ein Klettband, einen Kordelzug, einen Reißverschluss, einen elastisches Band oder einen Kleberverschluss einzusetzen. Bevorzugt kommt das Klettband zum Einsatz. Sofern der Kleberverschluss gewählt wird, ist es von Vorteil, wenn dieser temperaturabhängig reagiert, das heißt, dass ein Öffnen des Verschlusses erfolgt, sobald das Waschwasser eine bestimmte Temperatur erreicht hat.

Die Anordnung kann so getroffen sein, dass der Speicher als separate, in die Hülle einsetzbare, flüssigkeitsundurchlässige Messkappe ausgebildet ist. Als Messkappe kann beispielsweise die Verschlusskappe einer Flüssig-Waschmittelvorratsflasche dienen.

Nach einer anderen Ausführungsform der Erfindung ist es jedoch auch möglich, dass der Speicher von einer behälterartig ausgebildeten, flüssigkeitsundurchlässigen Folie gebildet ist. Vorzugsweise ist die Folie im Inneren der Hülle angeordnet. Wird der oben beschriebene, temperaturabhängige Kleberverschluss eingesetzt, so kann die Hülle insgesamt mit der Folie versehen sein. In diesem Falle würde erst nach Öffnung des Verschlusses ein Austreten des Wäschebehandlungsmittels - das flüssig oder auch pulverförmig sein kann - durch entsprechende Walkarbeit durch die Trommeldrehung der Maschine erfolgen.

Die Folie kann neben der Hülle eine separate Wandung bilden, so dass eine Zweistückigkeit vorliegt. Alternativ ist es nach einer Weiterbildung der Erfindung jedoch auch möglich, dass die Folie von einer Beschichtung oder Haut der Hülle gebildet ist. Mithin liegt dann hier eine Einstückigkeit vor.

Besonders vorteilhaft ist es, wenn die Folie im Bodenbereich der Hülle angeordnet ist und der Verschlussbereich der Hülle keine Folie aufweist. Mithin ist angrenzend an den Verschluss der Hülle ein folienfreier Bereich ausgebildet. Diese Ausgestaltung gestattet das Einfüllen des flüssigen Wäschebehandlungsmittels in dosierter Form in den Bodenbereich bzw. unteren Bereich der durch die Folie dann dort flüssigkeitsundurchlässigen Hülle. Nach dem Verschluss der Hülle wird diese dann zusammen mit der zu behandelnden Wäsche in die Trommel der Waschmaschine gegeben. Ein sofortiges Auslaufen der Wäschebehandlungsflüssigkeit ist vermieden, da der im Bereich des Verschlusses gelegene Abschnitt der Hülle, aufgrund seiner offenporigen Zellstruktur die oben beschriebene Rückhaltefähigkeit besitzt. Erst wenn während des Waschprozesses Wasser hinzutritt und eine Walkarbeit erfolgt, wird das Flüssig-Waschmittel oder dergleichen aus der erfindungsgemäßen Vorrichtung "ausgewaschen" und gelangt somit aktiv in den Waschprozess.

Eine weitere Variante zeichnet sich dadurch aus, dass die Hülle aus offenporigem Schaumstoff besteht. Die entsprechende Offenporigkeit erlaubt es, dass das Waschwasser in die Hülle gelangt und sich mit dem Wäschebehandlungsmittel des Speichers vermischt bzw. dieses löst, welche Lösung dann durch den offenporigen Schaumstoff hindurchtritt bzw. hindurchdiffundiert. Auf diese Weise erhält man entsprechend der Offenporigkeit über einen wählbaren Zeitraum eine kontinuierliche Abgabe bzw, ein kontinuierliches Austreten des Wäschebehandlungsmittels. Der offenporige Schaumstoff zur Bildung der Hülle gestattet es, entsprechende Formen zu erzeugen, um ein besonders gutes Unterbringen des das Wäschebehandlungsmittel aufnehmenden Speichers zu erhalten.

Auch sind - wie oben erwähnt - entsprechende Lösungen mit Schaumstoff und Folie oder be- bzw. verhautetem Schaumstoff möglich.

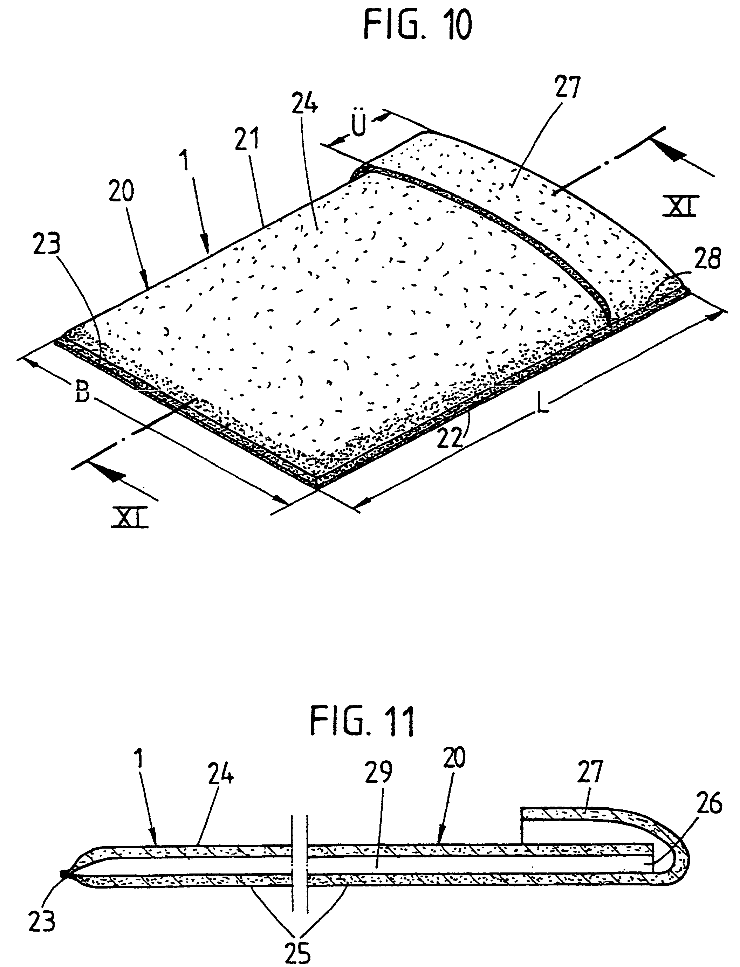

Es ist jedoch auch denkbar, dass der Speicher von einem verschließbaren Behältnis aus offenporigem Schaumstoff gebildet ist. Dadurch erhöht sich sogar sein Einsatzbereich. Er kann sowohl dazu dienen, einen Wäschebehandlungsflüssigkeit enthaltenden Behälter aufzunehmen als auch Waschpulver. Im letzten Falle kann der Behälter entfallen. Das entsprechende Schaumstoff-Behältnis kann in den verschiedensten Ausführungsformen erstellt werden, wie beispielsweise in Beutelform, Kuvertform (z.B. mit auf links gearbeitetem Hotelverschluss, wie dieser in Fig. 11 gezeigt ist). Das Einfüllen von Waschpulver in das Schaumstoff-Behältnis und Einlegen zwischen die Wäsche erfolgt in der vorbeschriebenen Weise. Nach dem Einlegen und Einschalten des Waschvorganges dringt das Waschwasser in den Innenraum des Behältnisses ein, vermischt sich mit dem Waschpulver, welche Lösung vorzugsweise nach einigen Minuten das Behältnis verlässt. Bedingt durch den Einsatz von Schaumstoff zur Schaffung des Behältnisses lassen sich die Herstellungskosten sehr niedrig halten.

Um den verschiedenen Anforderungen bei einem Waschprozess gerecht zu werden, ist der Schaumstoff bis mindestens 95° temperaturbeständig. Das Schaumstoff-Behältnis ist daher für jeden angestrebten Waschprozess geeignet.

Als dauerhaft und kochbeständig hat sich insbesondere offenporiger Polyurethan-Schaumstoff erwiesen. Die Dicke des Schaumstoffes zur Schaffung des Behältnisses liegt dabei zwischen 1 bis 6mm. In Versuchen hat sich als günstig erwiesen, eine Dicke von 3-4mm zu wählen. Vorzugsweise löst sich das Waschpulver in einem geschlossenen Beutel aus offenporigem Polyurethan in ca. 2 bis 3min. auf und gelangt dabei in das Waschwasser.

Diesbezüglich zeichnet sich eine vorteilhafte Bauform dadurch aus, dass die Hülle bzw. das Behältnis von einer Schaumstofftasche gebildet ist. Die Taschenform kommt einer einfachen, kostensparenden Herstellung entgegen. Auch eignet sie sich dazu, entweder Waschpulver oder einen Wäschebehandlungsflüssigkeit enthaltenden Behälter zu umhüllen.

Insbesondere bietet sich eine solche Bauform an, die dadurch gekennzeichnet ist, dass die Schaumstofftasche zwei rechteckförmige, deckend zueinander angeordnete, an drei Randseiten miteinander verbundene Taschenwände aufweist. Hierdurch ist ein weitgehend verlustfreier Zuschnitt zur Bildung der Schaumstofftasche möglich. Darüber hinaus ist die Verbindung an geradlinigen Randseiten vornehmbar.

Um ein ungewolltes Austreten des Tascheninhaltes vor der Durchflutung zu verhindern, geht von der einen Taschenwand eine umgelegte, die Taschenöffnung überfangende Verschlusslasche aus, deren Schmalkanten mit den entsprechenden Bereichen der Randseiten verbunden sind. Dies kann gleichzeitig bei der Randseitenverbindung geschehen. Insbesondere kommt einer günstigen Fertigung die Tatsache entgegen, dass die Randseiten zusammen mit der jeweils zugehörigen Schmalkante miteinander verschweißt sind. Zusätzliches Material zur Randseitenund Schmalkantenverbindung ist daher nicht erforderlich, da die Schmelzfähigkeit des Schaumstoffes ausgenutzt wird. Für die Randseitenverschweißung bietet sich insbesondere eine Hochfrequenzschweißung an.

Wenn die Verschlusslasche einstückig mit der zugehörigen Taschenwand ausgebildet ist, kann zur Bildung der Schaumstofftasche von einem einzigen Zuschnitt ausgegangen werden.

Gebrauchsvorteile ergeben sich dadurch, dass die Schaumstofftasche ein Format von etwa 20cm Länge und 15cm Breite aufweist. Daher kann sie genügend Waschmittel für einen Waschvorgang in einer Waschtrommel aufnehmen. Insbesondere bietet sich diese Form und Größe für eine Waschpulverdosierung an und könnte als sogenanntes "Waschmittelkissen" zum Einsatz kommen. Das Befüllen geschieht dabei über die an der einen Breitseite der Schamstofftasche ausgebildete Taschenöffnung.

Das Austreten des in der Schaumstofftasche befindlichen Waschpulvers durch die Taschenöffnung wird dadurch verhindert, dass die Verschlusslasche einen Überstand über die Taschenöffnung von etwa 3-6cm aufweist.

Die Zeichnungen veranschaulichen die Erfindung anhand mehrerer Ausführungsbeispiele und zwar zeigt:
- Fig. 1: eine aus offenporiger Zellstruktur bestehende Hülle zur Aufnahme einer Messkappe,
- Fig. 2: die Hülle gemäß Fig. 1 mit in ihrem Inneren aufgenommener Messkappe,
- Fig. 3: einen Längsschnitt entlang der Linie III-III in Fig. 2,
- Fig. 4: eine Draufsicht auf eine als kuvertartige Tasche ausgebildete Hülle,
- Fig. 5: eine Schnittansicht entlang der Linie V-V in Fig. 4,
- Fig. 6: eine Ansicht einer als Beutel ausgebildeten Hülle, deren Verschluss als Kordelzug ausgbildet ist,
- Fig. 7: eine Draufsicht auf ein weiteres Ausführungsbeispiel eines Beutels, der einen quadratischen Boden aufweist,
- Fig. 8: ein weiteres Ausführungsbeispiel einer Hülle, die im unteren Bereich mit einer flüssigkeitsundurchlässigen Folie versehen ist,
- Fig. 9: einen Querschnitt durch die Hülle gemäß Fig. 8 entlang der Linie IX-IX,
- Fig. 10: in perspektivischer Darstellung ein weiteres Ausführungsbeispiel einer in Form einer Schaumstofftasche ausgebildeten Hülle und
- Fig. 11: den Schnitt nach der Linie XI-XI in Fig. 10.

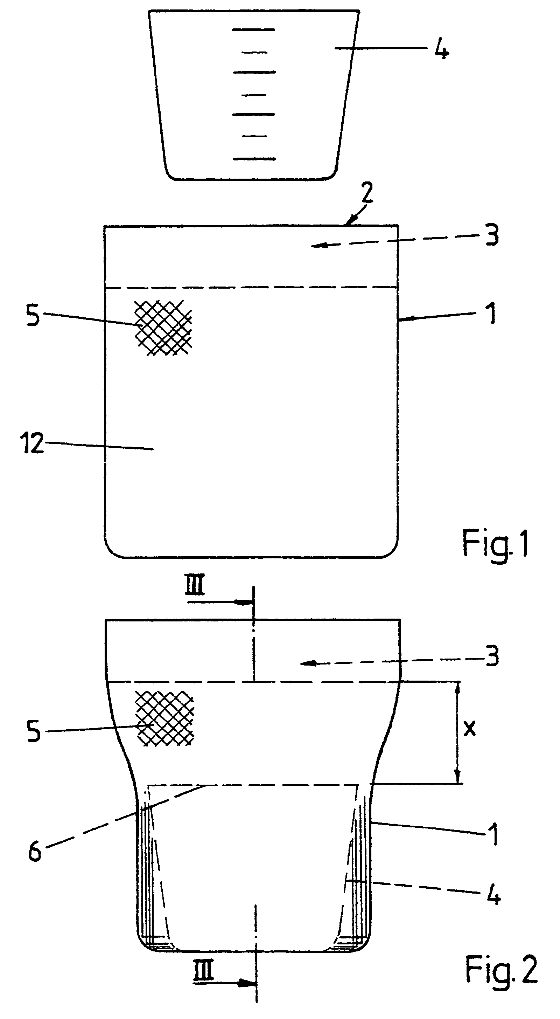

Die erfindungsgemäße Dosiervorrichtung besteht gemäß Fig. 1 aus einer Hülle 1, die eine Öffnung 2 aufweist, welche mittels eines Klettverschlusses 3 verschließbar ist. Die Abmessungen der Hülle 1 sind derart gewählt, dass durch die Öffnung 2 eine Messkappe 4 in das Innere der Hülle eingebracht werden kann (vgl. Fig. 2). Die Hülle besteht aus einer flexiblen, offenporigen Zellstruktur 5, die in Abhängigkeit von ihrem Material und ihrer Dicke die oben beschriebene Rückhaltefähigkeit besitzt. Als Zellstruktur kann Gewebe, Gewirke, Vlies, Schlingenware, z.B. Frottier oder Frottee, Strickware, Damast und vorzugsweise auch Schaumstoff zum Einsatz kommen.

Gemäß Fig. 2 ist sichergestellt, dass oberhalb des oberen Becherrandes 6 ein Abstand x bis zum Klettverschluss 3 besteht, mit anderen Worten eine Beutellänge - ohne Verschluss - ist größer als die Höhe der Messkappe 4. Hierdurch ist ein Austrittsfreiraum geschaffen.

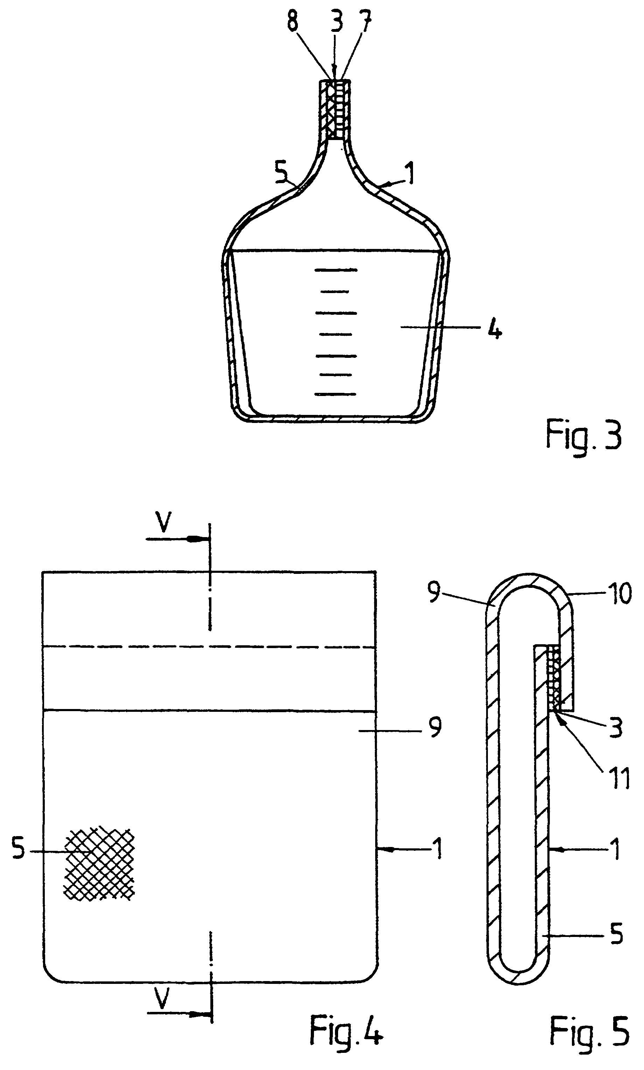

Die Fig. 3 verdeutlicht nochmal den Aufbau der erfindungsgemäßen Vorrichtung. Deutlich ist der aus zwei Bändern 7, 8 bestehende Klettverschluss 3 erkennbar.

Bei der Anwendung wird wie folgt vorgegangen:

Zunächst wird eine entsprechende Menge eines flüssigen Wäschebehandlungsmittels in die Messkappe 4 gegeben. Anschließend wird die Messkappe 4 durch die Öffnung 2 in das Innere der Hülle 1 eingebracht und schließlich die Öffnung 2 durch den Klettverschluss 3 verschlossen. Zusammen mit den zu behandelnden Wäschestücken wird dann die erfindungsgemäße Vorrichtung in die Trommel einer Waschmaschine oder dergleichen eingebracht. Durch die Trommelbewegung läuft die Wäschebehandlungsflüssigkeit aus der Messkappe 4 heraus, was insbesondere durch den mittels des Abstandes x gebildeten Freiraum möglich ist. Die offenporige Zellstruktur 5 der Hülle 1 saugt die Behandlungsflüssigkeit im Wesentlichen, zumindest jedoch teilweise, auf. Wenn nunmehr die Waschmaschine mit Wasser gefüllt ist, erfolgt durch die Walkbewegung ein Austreten bzw. Auswaschen des Flüssig-Waschmittels und wird damit für den Waschvorgang aktiviert. Nach Beendigung des Waschvorganges kann die erfindungsgemäße Hülle 1 wieder verwendet werden. Durch geeignete Materialauswahl von Messkappe 4 und Hülle 1 ist es möglich, die Vorrichtung auch selbst bei 95° Waschtemperatur einzusetzen. Wesentlich ist ferner, dass die zumeist etwas scharfkantige Messkappe keinen direkten Textilkontakt mit den zu behandelnden Wäschestücken hat, wodurch sich eine äußerst wäschefreundliche Behandlungsweise ergibt.

Die Fig. 4 zeigt ein weiteres Ausführungsbeispiel der erfindungsgemäßen Hülle 1, die gegenüber dem zuvor beschriebenen Ausführungsbeispiel nicht beutelförmig, sondern als kuvertartige Tasche 9 ausgebildet ist. Hierzu weist die Tasche 9 eine Überschlaglasche 10 auf, die mit einem Klettverschluss 3 zum Schließen der Tasche 11 festlegbar ist. Auch in diese Tasche 9 wird - wie zuvor beschrieben - ein entsprechender Messbecher eingebracht, der Einsatz erfolgt in gleicher Weise, wie zuvor beschrieben.

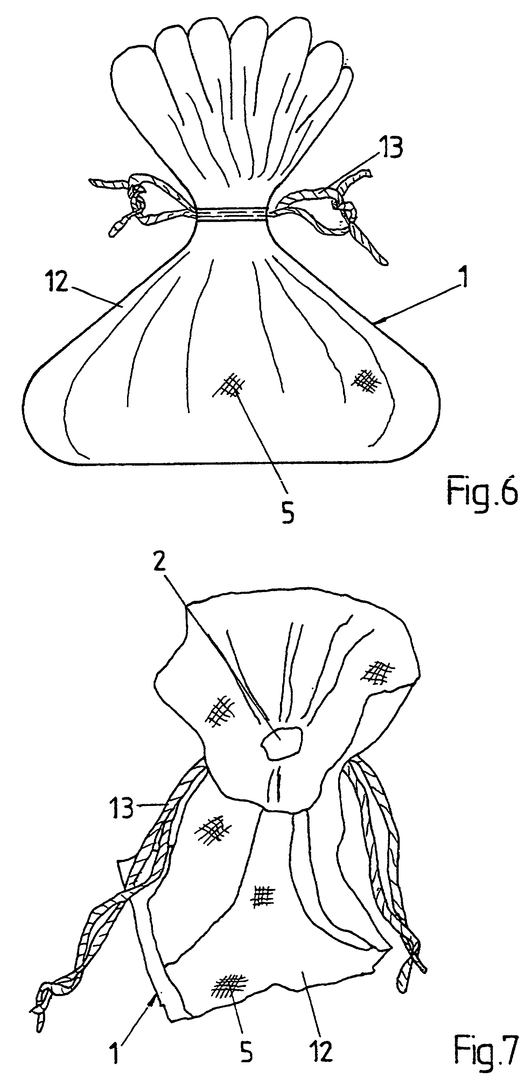

Die Figuren 6 und 7 zeigen ein weiteres Ausführungsbeispiel einer erfindungsgemäßen Vorrichtung, wobei die Hülle 1 als Beutel ausgebildet ist und als Verschluss einen Kordelzug 13 aufweist.

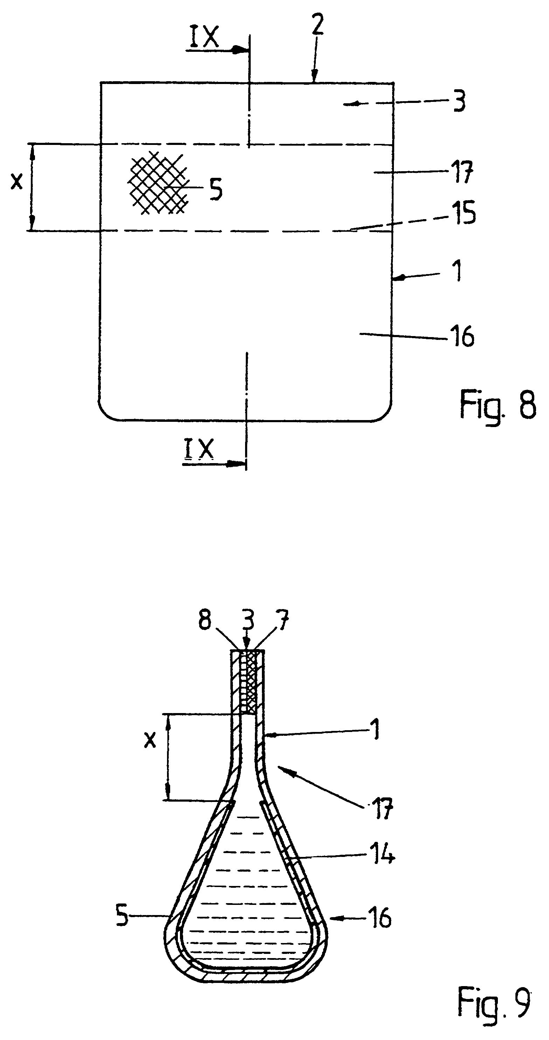

Während in den Ausführungsbeispielen der Figuren 1-7 der als Speicher für das flüssige Wäschebehandlungsmittel dienende Messbecher in das Innere der Hülle 1 eingebracht werden muss, ist nach den nun folgenden Ausführungsbeispielen der Figuren 8 und 9 dieser Einsetzvorgang nicht erforderlich. Der Speicher ist hier von einer behälterartig ausgebildeten, flüssigkeitsundurchlässigen Folie 14 gebildet, die an der Innenwand der beutelartigen Hülle 1 angeordnet ist. In dem dargestellten Ausführungsbeispiel ist die Hülle 1 "gemischtdurchlässig" gestaltet, das heißt, das Unterteil ist undurchlässig für die Wäschebehandlungsflüssigkeit, während das Oberteil durchlässig ist. Dieses folgt daraus, dass sich die Folie 14 nicht bis zu dem Klettverschluss 3 der Hülle 1 erstreckt, sondern hier den Abstand x belässt.

In der Fig. 8 deutet die gestrichelt eingezeichnete Linie 15 den Übergang von mit Folie 14 versehenem und folienfreiem Bereich der Hülle 1 an. Der Bodenbereich 16 ist - wie aus Fig. 9 ersichtlich - mit der Folie 14 versehen, während der an den Verschluss 3 angrenzende Bereich 17 keine Folie 14 aufweist, sondern nur aus der offenporigen Zellstruktur 5 besteht.

Für den Einsatz wird der Klettverschluss 3 der erfindungsgemäßen Vorrichtung geöffnet und anschließend das flüssige Wäschebehandlungsmittel in das Innere des Beutels gegossen. Dabei verhindert die Folie 14, dass es aus dem Boden der Vorrichtung austritt. Bei Verwendung entsprechend dünner oder durchscheinender Folien kann durch entsprechend aufgebrachte Markierungen der Dosiervorgang korrekt durchgeführt werden. Gegebenenfalls kann die Hülle 1 hierzu mit einem Sichtfenster versehen sein. Ist die Dosierung abgeschlossen, so wird der Klettverschluss 3 wieder verschlossen und die erfindungsgemäße Vorrichtung zusammen mit der zu behandelnden Wäsche in die Trommel einer Waschmaschine oder dergleichen gepackt. Durch die Trommelbewegung gelangt das flüssige Wäschebehandlungsmittel in den Bereich x der Hülle 1 und kann dort durch die offenporige, saugfähige Zellstruktur 5 nach und nach austreten.

Nach einem nicht dargestellten Ausführungsbeispiel ist es auch möglich, den gesamten inneren Bereich der Hülle 1 mit Folie 14 zu versehen, wobei dann der Verschluss der Hülle 1 derart ausgebildet sein muss, dass er ein Austreten des Wäschebehandlungsflüssigkeit zulässt. Dieses kann durch die Walkbewegung hervorgerufen werden, indem durch die hierdurch erfolgende Krafteinwirkung nach und nach der Hülleninhalt durch den nicht völlig dicht schließenden Verschluss herausgepresst wird. Wird ein temperaturabhängiger Kleber zum Schließen des Verschlusses eingesetzt, so öffnet dieser, sobald das Waschwasser die entsprechende Temperatur erreicht hat. Die Folie kann auch als Beschichtung der Hülle 1 ausgebildet werden. Zum Einsatz gelangen kann auch ein flexibler, weicher, kochbeständiger Kunststoff z.B. Santoprene oder Vestoprene.

Selbstverständlich liegt es im Rahmen der Erfindung, die Form der Hülle unterschiedlich zu gestalten. Ferner ist es möglich, die Hülle und gegebenenfalls auch die Folie mit Mengenmarkierungen, Gebrauchsanweisungen sowie Werbehinweisen und dergleichen zu bedrucken.

Ferner kann die Hülle so gestaltet sein, dass sich ein Zweitnutzen ergibt, der z.B. darin besteht, den Beutel als Waschhandschuh oder auch als Behälter für Seife zu benutzen.

Wird für die Hülle Schaumstoff verwendet, so bietet sich insbesondere solches Material an, welches bis mindestens 95° C temperaturbeständig ist. Vorzugsweise wird offenporiger Polyurethan-Schaum mit einer Dicke von 1-6mm verwendet. Einerseits kann in die Hülle aus offenporigem Schaumstoff ein entsprechender Speicher eingesetzt werden. Alternativ ist es auch möglich, dass der Speicher selbst durch ein verschließbares Behältnis aus offenporigem Schaumstoff gebildet ist. Ein solches Schaumstoff-Behältnis eignet sich zur Aufnahme von Waschpulver, ohne dass ein spezieller, das Wäschebehandlungsmittel aufnehmender Behälter vorgesehen sein müsste. Bei einer Schaumstoffdicke von 2mm löst sich das Waschpulver nach Einlegen des Behältnisses zwischen die Wäsche und Zugabe von Waschwasser in 2-3min. auf, wenn von offenporigem Polyurethan-Schaumstoff mit entsprechender Porengröße ausgegangen wird.

Gemäß der Darstellung in den Fig. 10 und 11 ist die Hülle 1 von einer Schaumstofftasche 20 gebildet. Als Material ist Polyurethan-Schaumstoff gewählt mit einer Dicke von ca. 3mm. Die Schaumstofftasche 20 weist zwei rechteckförmige, deckend zueinander angeordnete, an drei Randseiten 21, 22, 23 miteinander verbundene Taschenwände 24 und 25 auf. Von der rückwärtigen Taschenwand 25 geht eine umgelegte, die Taschenöffnung 26 überfangende Verschlusslasche 27 aus. Die Schmalkanten 28 sind mit den entsprechenden Bereichen der Randseiten 21, 22 verbunden. Die Verbindung der Randseiten zusammen mit der jeweils zugehörigen Schmalkante 28 erfolgt durch Verschweißung. Hierzu bietet sich insbesondere eine Hochfrequenzverschweißung an, wobei die entsprechenden Randbereiche der Schaumstofftasche miteinander verschmelzen.

Insbesondere geht aus Fig. 11 hervor, dass die Verschlusslasche 27 einstückig mit der zugehörigen Taschenwand 25 ausgebildet ist. Anstatt wie dargestellt ist, könnte sich diese rückwärtige Trennwand 25 einstückig in die vorderseitige Taschenwand 24 fortsetzen, so dass die Schaumstofftasche aus einem einzigen Zuschnitt gefertigt werden kann.

Beim Ausführungsbeispiel besitzt die Schaumstofftasche 20 ein rechteckiges Format von etwa 20cm Länge L, während die Breite B 15cm beträgt. Die Taschenöffnung 26 befindet sich an der der Randseite 23 gegenüberliegenden Breitseite der Schaumstofftasche. Als Überstand Ü der Verschlusslasche 27 über die Taschenöffnung 26 sind 4cm gewählt. Das Beschicken des Tascheninneren 29 erfolgt in der Weise, dass die vordere Taschenwand 24 über die Verschlusslasche 27 hinausgezogen wird unter Bildung einer Einfüllöffnung. Nach dem Einfüllvorgang ist dann die Verschlusslasche 27 in ihre überfangende Lage zu bringen, so dass eine Selbstentleerung des in die Schaumstofftasche eingebrachten Waschpulvers verhindert ist.

Die Schaumstofftasche 20 kann bei zurückgeklappter Verschlusslasche 27 als Waschhandschuh, z.B. für das Säubern von harten Oberflächen, benutzt werden. Die verschlossene Schaumstofftasche 20 lässt sich ferner im Nebennutzen als Wäschesäckchen für das Waschen empfindlicher Kleintextilien,z.B. Damenfeinstrümpfe oder Strumpfhosen, benutzen.

Alle offenbarten Merkmale sind (für sich) erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen.

## Patentansprüche

1. Verfahren zum Wäsche waschen in einer Waschmaschine, bei welchem man eine sackartige Vorrichtung verwendet, welche eine geschmeidige Hülle (1) aufweist, welche mit der gewünschten Menge eines eine bestimmte Aktivität im Verlaufe des Waschvorganges entfaltenden, teilchenförmigen Produktes gefüllt ist, wobei das Material, aus welchem diese Hülle (1) besteht, das Eindringen des wässrigen Waschmediums in den Sack ermöglicht, und wobei die Vorrichtung zusammen mit der Wäsche in die Waschmaschine eingebracht wird, **dadurch gekennzeichnet, dass** die Vorrichtung vom wiederverwendbaren Typus ist, und dass sie zu diesem Zweck eine Öffnung (2) aufweist, durch welche das Anfüllen des Sackes mit der gewünschten Menge des Produktes vor jedem Waschvorgang, bei welchem die Vorichtung verwendet wird, durchgeführt wird, wobei dieser Öffnung (2) Verschlussmittel (3, 13) zugeordnet sind, und wobei dieses Verfahren die folgenden verschiedenen Stufen umfasst:
- während die Öffnung offengehalten wird, füllt man den Sack durch diese Öffnung (2) mit der gewünschten Menge des im Verlaufe des Waschvorganges aktiven, teilchenförmigen Produktes;
- man wirkt auf die Verschlussmittel (3, 13) dahingehend ein, dass die Öffnung (2) verschlossen wird;
- man bringt die so verschlossene Vorrichtung (10, 30) zusammen mit der Wäsche in die Trommel der Waschmaschine ein;
- dann führt man den Waschvorgang in der üblichen Weise durch, wobei das teilchenförmige Produkt im Verlaufe des Waschvorganges in der Form einer wässrigen Lösung, welche hauptsächlich durch die Wände des Sackes, und zwar aus dem Inneren des Sackes nach dessen Außenseite hinaustritt, fortschreitend freigesetzt wird.

2. Verfahren nach Anspruch 1, **dadurch gekennzeichnet, dass** die Öffnung von Verschlussmitteln überfangen ist.

## Claims

1. A method for washing laundry in a washing machine, in which use is made of a sack-like arrangement which has a flexible enclosure (1) which is filled with the desired quantity of a particulate product which exhibits a specific activity during the washing operation, the material of which the enclosure (1) consists allowing the aqueous washing medium to penetrate into the sack, and the arrangement being introduced, together with the laundry, into the washing machine, **characterized in that** the arrangement is of the reusable type, and **in that**, for this purpose, it has an opening (2) through which the filling of the sack with the desired quantity of product before each washing operation in which the arrangement is used is effected, closure means (3, 13) being assigned to this opening (2), and this method comprising the following various steps:
- while the opening is held open, the sack is filled, through this opening (2), with the desired quantity of particulate product which is active during the washing operation;
- the opening (2) is closed by virtue of the closure means (3, 13) being acted on appropriately;
- the arrangement (10, 30) thus closed is introduced, together with the laundry, into the drum of the washing machine;
- the washing operation is then carried out in the customary manner, the particulate product being released continuously, during the washing operation, in the form of an aqueous solution which passes out predominantly through the walls of the sack, to be precise from the interior of the sack to the outside of the latter.

2. A method according to Claim 1, **characterized in that** the opening has closure means gripping over it.

## Revendications

1. Procédé pour laver du linge dans une machine à laver, dans lequel on utilise un dispositif du genre d'un sac présentant une enveloppe (1) souple, remplie de la quantité souhaitée d'un produit se présentant sous forme de particules, déployant une activité déterminée au cours du processus de lavage, le matériau dont se compose cette enveloppe (1) permettant la pénétration de l'agent de lavage aqueux dans le sac et le dispositif étant introduit conjointement avec le linge dans la machine à laver, **caractérisé en ce que** le dispositif est du type réutilisable, et qu'il présente à cette fin une ouverture (2), à travers laquelle est effectué le remplissage du sac avec la quantité souhaitée du produit, avant chaque processus de lavage pour lequel le dispositif est utilisé, à cette ouverture (2) étant associés des moyens de fermeture (3, 13) et ce procédé comprenant les différentes étapes suivantes :
- tandis que l'ouverture est laissée ouverte, on remplit le sac en passant par cette ouverture (2), avec la quantité souhaitée de produit se présentant sous forme de particules, et actif pendant le processus de lavage ;
- on agit sur les moyens de fermeture (3, 13) pour que l'ouverture (2) soit fermée ;
- on place le dispositif (10, 30) ainsi fermé, conjointement avec le linge, dans le tambour de la machine à laver ;
- on effectue alors le processus de lavage de la manière usuelle, le produit se présentant sous forme de particules, étant libéré progressivement au cours du processus de lavage sous la forme d'une solution aqueuse, qui, principalement, sort à travers par les parois du sac et, précisément, de l'intérieur du sac vers sa face extérieure.

2. Procédé selon la revendication 1, **caractérisé en ce que** l'ouverture est entourée de moyens de fermeture.
